(19) Europäisches Patentamt
European Patent Office
Office européen des brevets

(11) Veröffentlichungsnummer: **0 492 495 A2**

(12) **EUROPÄISCHE PATENTANMELDUNG**

(21) Anmeldenummer: **91121931.9**

(22) Anmeldetag: **20.12.91**

(51) Int. Cl.5: **C12N 11/08**, C08F 8/12, C08F 218/08, C12N 11/18, C12P 35/06

(30) Priorität: **24.12.90 DE 4041755**

(43) Veröffentlichungstag der Anmeldung: **01.07.92 Patentblatt 92/27**

(84) Benannte Vertragsstaaten: **AT BE CH DE DK ES FR GB GR IT LI LU NL SE**

(71) Anmelder: **HOECHST AKTIENGESELLSCHAFT
Postfach 80 03 20
W-6230 Frankfurt am Main 80(DE)**

(72) Erfinder: **Sauber, Klaus, Dr.
Königsteiner Strasse 144
W-6232 Bad Soden am Taunus(DE)**

(54) **Immobilisierte D-Aminosäureoxidase und dessen Verwendung zur Herstellung von Arzneimitteln.**

(57) Die Erfindung betrifft ein mit Enzymen beschichtetes Trägermaterial, das aus D-Aminosäureoxidase und einem Trägermaterial besteht. Der Träger ist ein vernetztes Copolymerisat, welches aus Vinylacetat- und/oder Vinylalkohol-Einheiten und Einheiten eines Vernetzungsmittels besteht. Der mit D-Aminosäureoxidase beschichtete Träger kann auch zusätzlich mit Katalase beschichtet sein. Das erfindungsgemäße Trägermaterial kann zur Herstellung von Arzneimitteln eingesetzt werden.

EP 0 492 495 A2

D-Aminosäure-Oxidase (im folgenden DAO) katalysiert die oxidative Desaminierung von D-Aminosäuren zu den entsprechenden -Ketosäuren, Ammoniak und Wasserstoffperoxid.

Neben der kommerziell erhältlichen DAO aus Schweinenieren wird das Enzym von Bakterien, Hefen und Pilzen synthetisiert. Unter diesen zeichnet sich Trigonopsis variabilis als potentester DAO-Produzent aus. Neben der Verwendung dieses Enzyms zur Racemat-Trennung von D,L-Aminosäuren und dem quantitativen Nachweis von D-Aminosäuren in verschiedenen Lösungen ist die Fähigkeit zur oxidativen Desaminierung von Cephalosporin C besonders hervorzuheben. Diese katalytische Eigenschaft wird zur Herstellung von α-Ketoadipinyl-7-aminocephalosporansäure und Glutaryl-7-aminocephalosporansäure benutzt, welche anschließend durch eine Acylase zu 7-Aminocephalosporansäure umgesetzt werden kann.

In der Deutschen Offenlegungsschrift 22 19 454 (US-Patentschrift 3 801 458) wird die Umsetzung von Cephalosporin C-Derivaten mit Hilfe von aktivierten Zellen von Trigonopsis variabilis CBS 40 95 beschrieben. "Aktiviert" bedeutet hier, daß die Hefezellen einem physikalischen und/oder chemischen Verfahren unterzogen wurden, so daß die in den Zellen enthaltene DAO zur Katalyse der Oxidation von Cephalosporin C zugänglich wird, jedoch nicht substantiell freigesetzt wird.

In der Patentanmeldung WO 86 04 087 wird die Reinigung und Immobilisierung von DAO aus Trigonopsis variabilis beschrieben und dessen Verwendung zur oxidativen Desaminierung von Cephalosporin C. Es werden jedoch keine Angaben zur operationellen Stabilität gemacht und die Bindungsausbeute wird mit 40 % angegeben.

Aufgabe der vorliegenden Erfindung war es die Stabilität der D-Aminosäureoxidase zu verbessern. Überraschenderweise wurde nun gefunden, daß durch die Koppelung von D-Aminosäureoxidase an ein Trägermaterial aus einem vernetzten Copolymerisat, das im wesentlichen aus Vinylacetat- und/oder Vinylalkohol-Einheiten und Einheiten eines Vernetzungsmittels besteht, ein Komplex entsteht, bei dem das Enzym DAO lange Zeit seine Enzymaktivität behält.

Die Erfindung betrifft somit ein mit Enzymen beschichtetes Trägermaterial, das D-Aminosäureoxidase und ein poröses, perlförmiges Trägermaterial enthält, wobei das Trägermaterial aus einem vernetzten Copolymerisat, das im wesentlichen aus Vinylacetat- und/oder Vinylalkohol-Einheiten und Einheiten eines Vernetzungsmittels besteht, wobei die Einheiten des Vernetzungsmittels einpolymerisierte Verbindungen der allgemeinen Formeln

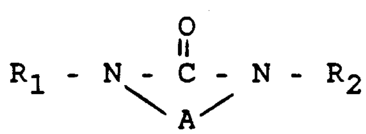

(I)

und/oder

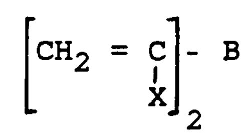

(II)

sind, wobei $R_1$, $R_2$ in Formel (1) gleich oder verschieden sein können und Vinyl-, 1-Acyloxy-Vinyl-, Allyl- oder 2-Acyloxy-Allyl- bedeuten, A einen zweiwertigen Kohlenwasserstoffrest mit 2 bis 8 C-Atomen darstellt, B in Formel (II) für einen zweiwertigen Kohlenwasserstoffrest mit 1 bis 8 C-Atomen steht und X Acyloxy bedeutet, wobei die Acyloxygruppe ein Rest mit 2 bis 18 C-Atomen ist, die Menge an Vernetzungsmittel-Einheiten 1 bis 60 Gew.-%, bezogen auf das Polymere, beträgt, und die Acyloxy-Gruppen der Vinylacylat-Einheiten als solche vorliegen oder teilweise oder vollständig zu Hydroxylgruppen verseift sind, und die mittlere Teilchengröße der Perlen 20 bis 800 µm und der mittlere Porendurchmesser 2 bis 10 000 nm beträgt.

Es war überraschend, daß die Enzyme in einer immobilisierten Form nach der Kopplung an den genannten Träger eine Lagerstabilität von mindestens 6 Monaten zeigen. Im Vergleich zu anderen bekannten Enzymträgern wie z.B. ®Eupergit (Röhm), Vinyl-®Sepharose (Kem-en-tec) BrCn-aktivierte-®Sepharose (Pharmacia) zeigte der erfindungsgemäße Träger mit der DAO eine überraschend hohe Bindungsausbeute, sowie eine längere operationelle Stabilität. Besonders bevorzugt werden der Vinylacetat-Epoxy-Träger von Riedel de Haen eingesetzt, beispielsweise VA-Epoxy-®Biosynth.

Die Herstellung der eingesetzten porösen, perlförmigen Trägermaterialien ist aus der DE-OS 33 44 912 bekannt, womit hierauf Bezug genommen wird.

Die Verankerungsreaktion zwischen den erfindungsgemäß eingesetzten Enzymen und dem Trägermaterial wird in bekannter Weise durchgeführt, wie etwa in der DE-OS 24 07 340 oder in den DE-Patentschriften 22 15 687, 24 21 789 und 25 52 510 beschrieben.

Die Erfindung bezieht sich auch auf die Verwendung des erfindungsgemäßen Trägermaterials für die oxidative Desaminierung von Cephalosporin C-Derivaten.

Unter dem Begriff Cephalosporin C-Derivate werden beispielsweise Verbindungen wie die der Formel III

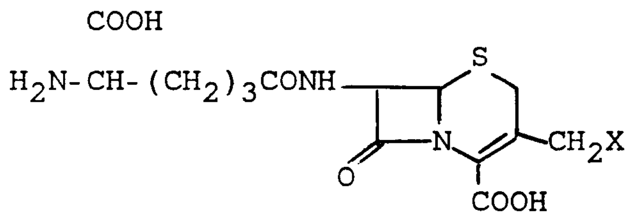

(III)

in welcher

X für eine Acetatgruppe,
den Rest eines Nukleophils,
einen Heterozyklus,
eine Hydroxygruppe oder
Wasserstoff steht,
sowie Salze davon verstanden.

Cephalosporin C steht für eine Verbindung der Formel III

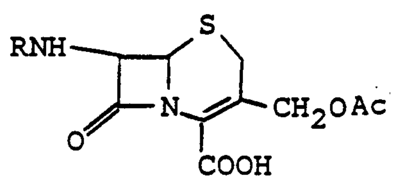

(III)

worin R

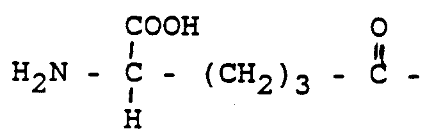

bedeutet.

α-Ketoadipinyl-7-aminocephalosporansäure ist eine Verbindung der Formel II worin R

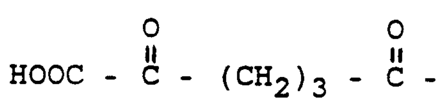

bedeutet.

Glutaryl-7-aminocephalosporansäure ist eine Verbindung der Formel II worin R

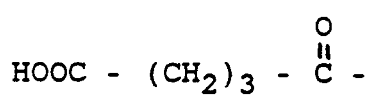

bedeutet.

7-Aminocephalosporansäure ist eine Verbindung der Formel II worin R Wasserstoff bedeutet.

Ferner betrifft die Erfindung ein Verfahren zur Herstellung der beschichteten Trägermaterialien, das dadurch gekennzeichnet ist, daß eine D-Aminosäureoxidase-haltige Lösung mit einem porösen, perlförmigen Trägermaterial aus einem vernetzten Copolymerisat, das im wesentlichen aus Vinylacetat- und/oder Vinylalkohol-Einheiten und Einheiten eines Vernetzungsmittels besteht, wobei die Einheiten des Vernetzungsmittels einpolymerisierte Verbindungen der allgemeinen Formeln

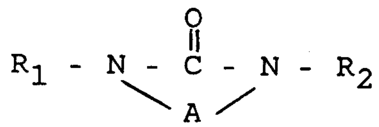

(I)

und/oder

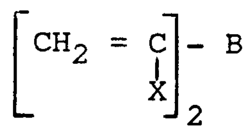

(II)

sind, wobei $R_1$, $R_2$ in Formel (I) gleich oder verschieden sein können und Vinyl-, 1-Acyloxy-Vinyl-, Allyl- oder 2-Acyloxy-Allyl- bedeuten, A einen zweiwertigen Kohlenwasserstoffrest mit 2 bis 8 C-Atomen darstellt, B in Formel (II) für einen zweiwertigen Kohlenwasserstoffrest mit 1 bis 8 C-Atomen steht und X Acyloxy bedeutet, wobei die Acyloxygruppe ein Rest mit 2 bis 18 C-Atomen ist, die Menge an Vernetzungsmittel-Einheiten 1 bis 60 Gew.-%, bezogen auf das Polymere, beträgt, und die Acyloxy-Gruppen der Vinylacylat-Einheiten als solche vorliegen oder teilweise oder vollständig zu Hydroxylgruppen verseift sind, und die mittlere Teilchengröße der Perlen 20 bis 800 µm und der mittlere Porendurchmesser 2 bis 10 000 nm beträgt inkubiert wird.

Die Vinylacetat-Einheiten des Trägerpolymerisats enthalten vorzugsweise 2 bis 18 C-Atome, insbesondere 2 bis 6 C-Atome im Acylatrest. Bevorzugt ist dies der Acetat- oder Propionatrest. Es können auch verschiedene Acylatreste im Polymerisat vorhanden sein, d.h. zu seiner Herstellung können auch Gemische der entsprechenden Vinylacylate eingesetzt werden.

In dem Vernetzungsmittel gemäß der Formel (I) stellt A bevorzugt einen verzweigten oder unverzweigten aliphatischen Kohlenwasserstoffrest mit 2 bis 5 C-Atomen, insbesondere 2 oder 3 C-Atomen dar. Besonders bevorzugt ist dies der Ethylen- oder Propylenrest. Falls $R_1/R_2$ dieser Formel (1) für 1-Acyloxy-Vinyl- oder 2-Acyloxy-Allylstehen, so enthält die Acyloxy-Gruppe darin vorzugsweise 2 bis 18 C-Atome, insbesondere 2 bis 6 C-Atome. Bevorzugt bedeutet Acyloxy den Acetat- oder Propionatrest. Vorzugsweise haben die Reste $R_1/R_2$ die Bedeutung von Vinyl. Eine bevorzugte Vernetzer-Einheit in dem erfindungsgemäß eingesetzten Polymerisat leitet sich dementsprechend von N,N'-Divinyl-ethylen-harnstoff ab. Dieser Vernetzer bewirkt eine besonders hydrolysebeständige Verknüpfung. Ein weiterer bevorzugter Vertreter ist N,N'-Divinylpropylenharnstoff.

In dem Vernetzungsmittel gemäß der Formel (II) hat B bevorzugt die Bedeutung eines zweiwertigen Kohlenwasserstoffrestes, insbesondere eines verzweigten oder unverzweigten Alkylenrestes mit 2 bis 6 C-Atomen, vorzugsweise die gleiche Bedeutung wie oben für die Reste $R_1$ und $R_2$ in der Formel (I) beschrieben. Ein bevorzugter Vernetzer dieser Art ist beispielsweise 3,3-Dimethyl-pentadien-2,4-diacetat, das besonders leicht mit dem Vinylacetat copolymerisiert.

Die Menge an Einheiten des Vernetzungsmittels (II) beträgt im allgemeinen 0 bis 100 %, insbesondere 0 bis 60 %, bezogen auf die Gesamtmenge an Vernetzer-Einheiten im Polymerisat.

Die Gesamtmenge an Vernetzer-Einheiten in dem Träger-Polymerisat liegt in den beanspruchten Bereichen und hängt von der für den jeweiligen Anwendungszweck gewünschten Vernetzungsdichte ab. So ist beispielsweise bei der Anwendung als Trägermaterial für Enzymreaktionen im Rührkessel oder für Diagnostika eine relativ geringe Vernetzerdichte vorteilhaft, was einen niederen Gehalt an vernetzenden Monomer-Einheiten zur Voraussetzung hat. Vernetzergehalte unterhalb von 0,1 Gew.-% führen in den meisten Fällen zu nicht mehr brauchbaren Produkten. Als unter Grenze kann daher im allgemeinen etwa 1 Gew.-% angegeben werden. Vernetzergehalte oberhalb von 60 Gew.-% sind grundsätzlich möglich, erbringen in der Regel jedoch keine weiteren Vorteile.

Je nach Anwendungszweck liegt die Menge an Vernetzer-Einheiten vorzugsweise bei 1 bis 50 Gew.-%

und insbesondere bei 1 bis 40 Gew.-%, bezogen auf das Polymere. Bei dem erfindungsgemäßen Einsatz als Trägermaterial für DAO liegt die Untergrenze bevorzugt bei 2,5 Gew.-% und besonders bevorzugt bei 10 Gew.-%. Falls nur Vernetzer-Einheiten gemäß Formel (II) vorliegen, so beträgt deren Untergrenze bevorzugt 2,5 Gew.-%.

Es kann von Vorteil sein, wenn das Träger-Polymerisat zusätzlich noch Monomer-Einheiten eines mit Vinylacetat copolymerisierbaren Monomeren enthält, wobei deren Menge im allgemeinen 10 Gew.-%, bezogen auf das Gesamtpolymere, nicht überschreitet und vorzugsweise zwischen 0,1 und 5 Gew.-% liegt. Beispiele für derartige Monomere, die gegebenenfalls im Gemisch eingesetzt werden können, sind: N-Vinylpyrrolidon, Vinylencarbonat, (Meth)acrylsäure, (Meth)acrylnitril, (Meth)acrylamid, (Meth)-acrylsäurealkylester mit jeweils 2 bis 12 C-Atomen, vorzugsweise 2 bis 4 C-Atomen im Alkylrest, Hydroxylalkylester der (Meth)acrylsäure mit 2 bis 6 C-Atomen in der Alkylgruppe, N-Vinyl-N-alkylacetamid, Styrol, $\alpha$-Methylstyrol und dergleichen.

Das vernetzte Träger-Polymerisat liegt vorzugsweise in Form von Perlen vor, die überwiegend kugelförmige Gestalt aufweisen, deren mittlere Teilchengröße im trockenen, ungequollenen Zustand 20 bis 800 $\mu$m, vorzugsweise 50 bis 300 $\mu$m beträgt und die vorzugsweise eine enge Teilchengrößenverteilung aufweisen. Das jeweilige Optimum der Teilchengröße hängt dabei vor allem von dem speziellen Einsatzgebiet ab. Bei einem ohne Druck durchgeführten Säulenverfahren wird man beispielsweise die Teilchengröße innerhalb der vorstehend genannten Grenzen entsprechend größer wählen als bei einem Druck-Verfahren. Die Perlen des erfindungsgemäß eingesetzten Polymerisates sind überwiegend makroporös ausgebildet. Der mittlere Porendurchmesser liegt im allgemeinen im Bereich von 2 bis 10000 nm, vorzugsweise 5 bis 200 nm und insbesondere 20 bis 200 nm.

Die Acylat-Gruppen der Vinylacylat-Einheiten im erfindungsgemäß eingesetzten Polymerisat liegen als solche vor oder sind vorzugsweise teilweise oder vollständig zu OH-Gruppen verseift. Dabei sind zumindest 10 Gew.-% der Acyloxy-Gruppen durch Hydroxyl-Gruppen ersetzt. Der Verseifungsgrad beträgt jedoch im allgemeinen mehr als 50 %, vorzugsweise mehr als 70 % und insbesondere 90 bis 100 %. In dem durch Verseifung erhaltenen vernetzten Polymeren (Polyvinylalkohol) ist vorzugsweise zumindest ein Teil der OH-Gruppen durch sogenannte "Spacer"-Gruppen besetzt (bezüglich "Spacer" siehe weiter unten).

Das Copolymerisat in Form des Polyvinylacetatgels ist nicht hydrophil; zur Anwendung in Wasser muß die Estergruppe hydrolysiert werden. Das kann in bekannter Weise alkalisch durch Quellen des Produktes in einem Alkohol, z.B. Methanol und Zugabe von wäßrigem Alkali wie Natronlauge geschehen oder durch Umesterung des alkoholgequollenen Produktes mit katalytischen Mengen Säure oder Base bei laufender z.B. destillativer Entfernung des gebildeten Esters (vgl. DE-PS 15 17 935). Die Verseifung kann auf jeder beliebigen Stufe abgebrochen werden, so daß je nach Verwendungszweck der Grad der Hydrophilie des Gels eingestellt werden kann.

Beim Einsatz des perlförmigen vernetzten Polyvinylalkoholgels als Träger für die DAO, die durch eine kovalente Bindung an den Träger fixiert werden soll, ist es in vielen Fällen zweckmäßig, das Gel zuvor mit sogenannten "Spacern" zu modifizieren. Unter "Spacer" versteht man dabei Verbindungen, die sowohl mit dem Trägerpolymeren als auch mit der biologisch aktiven Substanz reagieren und zwischen beiden gewissermaßen eine Brücke bilden. Die Umsetzung des Perlpolymerisats mit dem Spacer kann entweder direkt oder vorzugsweise nach vorheriger Verseifung der Acylatgruppen erfolgen. Der Umsatzgrad hängt dabei u.a. von der Sperrigkeit des Spacers und der Zugänglichkeit der Acylatgruppe bzw. der daraus entstandenen sekundären Hydroxylgruppen ab. Als Spacer kommen erfindungsgemäß die hierfür bekannten homo- und heterobifunktionellen Verbindungen in Frage, deren zweite funktionelle Gruppe die Kopplung mit der zu fixierenden biologisch aktiven Substanz übernimmt (vgl. die DE-Patentschriften 24 21 789 und 25 52 510, sowie Ullmanns Encyclopädie der technischen Chemie, 4. Auflage, Bd. 10, Seite 540 und "Characterization of Immobilized Biocatalysts", Verlag Chemie, Weinheim, 1979, S.53).

Bei den eingesetzten Spacern handelt es sich beispielsweise um Verbindungen, welche die nachstehenden Gruppen einführen:

$-(CH_2)_n-NH_2$; n = 2-12

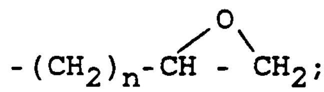

n = 1-8

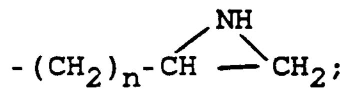

n = 1-8

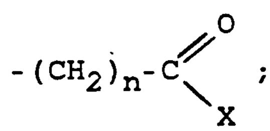

n = 1-8
X = H, OH, Halogen $N_3$, OR

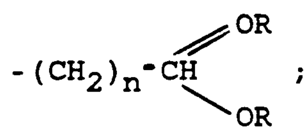

n = 1-6
R = Alkylrest mit 1-6 C-Atomen

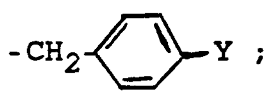

Y = $NH_2$, $N_2$, NCO

Bevorzugte "Spacer" sind solche, die hydrolysebeständige chemische Verbindungen herbeiführen, wie Epichlorhydrin oder dessen Homologe ($\alpha,\beta$-Epoxy-$\omega$-halogenalkane). Die Umsetzung der Polyvinylalkohole (Polivinylacylate) erfolgt dabei ohne Lösungsmittel oder in Gegenwart eines Lösungsmittels, vorzugsweise in Anwesenheit eines Katalysators. Die Umsetzungsdauer liegt - abhängig von der Temperatur, die zwischen Raumtemperatur und Rückflußtemperatur des Epichlorhydrins (113-115° C) liegen kann - im allgemeinen zwischen 30 Minuten und 24 Stunden. Als Katalysator kommen z.B. NaOH (in Pulverform) oder wäßrige Alkalien, Dimethylformamid, Triethylamin und andere Säureakzeptoren in Frage.

Die Umsetzung zwischen der DAO und dem Trägermaterial erfolgt zwischen 0 und +40° C, bevorzugt bei Raumtemperatur. Die Verankerungsreaktion erfolgt vorzugsweise in der Nähe eines neutralen pH-Wertes, beispielsweise bei pH-Werten von 5 bis 9, vorzugsweise in Gegenwart von Phosphatpuffern, mit einer Ionenstärke von 0,5 bis 1,5 M.

D-Aminosäureoxidase kann beispielsweise aus Schweinenieren, Bakterien, Hefen oder Pilzen gewonnen werden. Bevorzugt wird die DAO aus der Hefe Trigonopsis variabilis CBS 4095 verwendet. Für die Koppelung an den Enzymträger können gereinigte, teilweise gereinigte oder rohe DAO-haltige Zellextrakte eingesetzt werden. Die Reinigung von DAO kann nach klassischem Verfahren z.B. über Ammoniumsulfat-Fällung, Ionenaustauscher- oder Gelpermeationschromatographie erfolgen. Bevorzugt werden DAO-haltige Enzymlösungen verwendet die nach ®DEAE-Cellulose Ionenaustauscherchromatographie erhalten werden.

Ferner kann mit der DAO auch Katalase aus dem Enzymträger gekoppelt werden. Die Katalase kann beispielsweise von aus Tieren, Bakterien, Hefen oder Pilzen gewonnen werden. Bevorzugt wird die Katalase aus der Hefge Trigonopsis variabilis CBS 4095 gewonnen. Für die Kopplung an den Enzymträger können gereinigte, teilweise gereinigte oder rohe Katalase-haltige Zellextrakte verwendet werden. Die Katalase kann gleichzeitig mit der DAO, vor oder nach der DAO an den Enzymträger gekoppelt werden. Es können auch Enzymträger die nur mit DAO oder nur mit Katalase beschichtet sind gemischt werden.

Im folgenden wird die Erfindung anhand von Beispielen näher erläutert. Prozentangaben beziehen sich auf das Gewicht.

Beispiel 1

Die Kulturen von Trigonopsis variabilis CBS 4095 wurden zuerst in geschüttelten Kolben und anschließend in gerührten Fermentern herangezogen, die das von Sentheshanmuganathan und Nickerson (J. Gen.

Microbiol. 27, 465, 1962) beschriebene Medium, mit entweder Methionin oder Alanin als Stickstoffquelle, enthalten.

Zur DAO-Bestimmung werden 0,4 g Zellen eingefroren, gefolgt von Auftauen bei saurem pH, z.B. etwa pH 3-4; das Frieren kann bei einer Temperatur unter -10 °C, z.B. etwa -20 °C durchgeführt werden. Es sollte genügend lange gefroren werden, um die Permeabilisierung der Zellen hervorzurufen, z.B. zumindest 1 Stunde bei -20 °C.

Die Aktivität wird mit folgendem Testansatz photometrisch bestimmt:

| Lösungen: | |
|---|---|
| 1) Puffer | 100 mM KPP; pH 7,3; luftgesättigt |
| 2) o-Phenylendiamin | 0.02 % in $H_2O$ |
| 3) Peroxidase | 1 mg/ml in Puffer |
| 4) Enzym oder permeabilisierte Zellen | Optimal: 0,5 - 1,0 Units/ml |
| 5) Substrat | 150 mM Na - CPC (100 %) in Puffer |

Testdurchführung:

$\lambda$ = 405 nm (Maximum)
$\epsilon$ = 4020 l/mol*cm
$\nu$ = 30 °C

| Volumen: | Endkonzentration: |
|---|---|
| 1) 2.00 ml | 83 mM |
| 2) 0.50 ml | 0.0034 % |
| 3) 0.10 ml | 0.034 mg/ml |
| 4) 0.05 ml | |
| 2 min warten | |
| 5) 0.30 ml | 15.25 mM |
| 2.95 ml | |

Berechnung:

$$\frac{\text{Units}}{\text{ml}} = \frac{\Delta E * \text{Verdünnung} * \text{Gesamtvolumen}}{\text{min} * \epsilon * d * \text{Probenvolumen}}$$

Unter den obengenannten Bedingungen wird eine Enzymaktivität im Fermenter von 200 U/l erreicht.

Bei den Aktivitätsbestimmungen mit immobilisiertem Enzym werden diskontinuierlich Proben gezogen und in der HPLC die Abnahme der CPC-Konzentration bestimmt. Die mobile Phase besteht aus 40 mM Kaliumphosphat-Puffer (pH 4,3) und 20 % MeOH mit 10 mg/l Tetrabutylammoniumhydrogensulfat. Die stationäre Phase aus ®Lichrospher 100 RP 18 (5 µm).

Beispiel 2 Synthese der Träger

a) Suspensionspolymerisation

In einem 1,4 l Glaskolben mit Rührer, Rückflußkühler und Thermometer wurde unter Stickstoffatmosphäre eine organische Phase, bestehend aus einer Lösung von 60,0 g Vinylacetat, 40,0 g N,N'-Divinylethylenharnstoff, 80,0 g 2-Ethylhexanol, 20,0 g Polyglykol B 11/50 (Hoechst AG) und 2,0 g Azoisobutyronitril ®Porofor N (Bayer AG) unter Rühren in einer wäßrigen Phase, bestehend aus 3,2 g $Na_2HPO_4$, 8,0 g Polyvinylpyrrolidon (MG ca. 360 000) und 800 ml Wasser suspendiert. Durch Erwärmen auf 75 °C im

Heizbad wurde die Polymerisation gestartet. Nach zwei Stunden wurde die Temperatur auf 85 ° C erhöht und nach zwei weiteren Stunden die Polymerisation beendet. Die erhaltene Suspension wurde auf 25 ° C abgekühlt, abgesaugt und viermal mit je 1 l Wasser, dreimal mit je 1 l Methanol und zweimal mit je 1 l Aceton während 30 Minuten verrührt, abgesaugt und bei 50 ° C und 200 Torr über Nacht im Vakuumtrokkenschrank unter Stickstoff getrocknet. Die Ausbeute betrug 75 g. Es fielen trübe Perlen mit glänzender Oberfläche an. Das Schüttgewicht betrug 300 g/l. Daraus ergibt sich ein Schüttvolumen von 3,3 ml/g.

b) Partielle Verseifung

Es wurden 50 g trockenes Produkt zusammen mit 150 ml Methanol und einer Lösung von 17,5 g NaOH in 150 ml Wasser während 3 Stunden bei 30 ° C verrührt, abgesaugt, in 500 ml Methanol mit Essigsäure neutralisiert, einmal mit 500 ml Methanol und zweimal mit je 500 ml Aceton verrührt, abgesaugt, gesiebt und getrocknet. Die Ausbeute betrug 34,4 g (= 68,8 Gew.-%) bezogen auf Polymerisat. Die Perlen waren trübe und hatten eine glänzende Oberfläche. Das Schüttgewicht betrug 353 g/l (errechnetes Schüttvolumen 2,8 ml/g).
Siebverteilung: > 300 µm 19,0 g (55,2 Gew.-%), 200-300 µm 8,9 g (25,9 Gew.-%), 100-200 µm 6,1 g (17,9 Gew.-%) und 50-100 µm 0,4 g (1,2 Gew.-%). Verseifungsgrad (IR, bezogen auf Mol): 73 %.

c) Anlagerung des Spacer

Es wurden 10,0 g der trockenen Siebfraktion 50-200 µm 4 Stunden in 100 ml Epichlorhydrin bei 25 ° C gequollen, dann 4 Stunden unter schwachem Rühren auf 115 ° C erhitzt und nach dem Abkühlen auf 25 ° C abgesaugt. Dann wurde zweimal mit jeweils 200 ml Aceton während 30 Minuten verrührt, abgesaugt und über Nacht im Trockenschrank unter vermindertem Druck bei 50 ° C unter Stickstoffüberlagerung aufbewahrt.
Die Auswaage ergab 9,8 g trockenes Produkt mit einem Schüttgewicht von 315 g/l und einem Epoxidäquivalent von 350 µmol/g.

Beispiel 3

10 g Feuchtzellen hergestellt wie in Beispiel 3 mit einer Aktivität von 30 U/g werden zu gleichen Gewichtsteilen mit 20 mM Kaliumphosphatpuffer, pH 8,0, versetzt und suspendiert. Die Mischung wird unter Kühlung in einer Dyno-Mühle bei einer Verweilzeit von 3 x 5 min gemahlen. Die Aktivitätsausbeute im Überstand nach Zentrifugation bei 13000 g beträgt 60 bis 80 %; im Mittel sind das 210 U. Der leicht trübe Rohextrakt wird gegen einen 20 mM Kaliumphosphatpuffer, pH 8,0, dialysiert.
Danach wird soviel DEAE-Cellulose von Whatman zugegeben, daß die DAO vollständig bindet. Anschließend wird das gebundene Enzym in eine Säule gefüllt und die DAO mit steigender Ionenstärke (0-0,5 M NaCl) eluiert. Die aktiven Fraktionen werden vereinigt, über Ultrafiltration konzentriert und mit 1 M Kaliumphosphatpuffer (pH = 8.0) umgepuffert. Im Durchschnitt enthält die so gereinigte DAO-Lösung 25 U/ml.

Beispiel 4

5 ml einer nach Beispiel 3 erhaltenen Lösung werden auf 1 g VA-Epoxy Biosynth® von Riedel-deHaen gegeben und 3 Tage bei Raumtemperatur verschlossen stehengelassen. Das Enzym bindet in dieser Zeit kovalent an den Oxirangruppen-haltigen Träger. Danach wird das immobilisierte Enzym mit 1 M NaCl-Lösung gewaschen. Die Bindungseffizienz beträgt im Mittel 0,83. Geringfügige Mengen sind im Waschwasser. Das immobilisierte Enzym hat ca. 32 U/g (Feuchtgewicht, Fgw). Die Lagerung erfolgt in einem 20 mM Kaliumphosphat-Puffer, der 0,02 % Natriumazid enthält, bei 4 Grad Celsius.

Beispiel 5

Wie Beispiel 2, jedoch wird als Träger Eupergit® von Röhm, Darmstadt, eingesetzt. Ergebnis in Tabelle 1.

Beispiel 6

2 ml DAO-Lösung werden gegen 50 mM Kaliumphosphatpuffer (pH = 8,6) dialyisert. Die umgepufferte

Lösung mit insgesamt 60 U wird zur Fixierung bei Raumtemperatur (R.T). 18 h lang mit 2 ml Vinylsepharose-Suspension (60 %) inkubiert. Es werden 1,2 ml Sepharose erhalten mit 22 U/ml (s. Tabelle 1), was 43 % Bindungsausbeute entspricht. Im Waschwasser sind 10 U, so daß sich ein $\eta$ von 0,51 errechnet.

Beispiel 7

1 ml DAO-Lösung mit 3,2 U, die gegen 0,5 M Kaliumphosphatpuffer, pH 8,7, dialysiert worden waren, werden zu 0,2 g CNBr-aktivierter Sepharose (Pharmacia) bei R.T. gegeben. Nach 90 min wird die Kopplung beendet und nach Herstellervorschrift gewaschen. Überzählige Bindungsgruppen werden mit Glycin inaktiviert. Ergebnis s. Tabelle 1.

Beispiel 8

Das wie in Beispiel 4 immobilisierte Enzym wird unter den in Beispiel 4 genannten Bedingungen gelagert und jeden Monat wurde die Aktivität gemessen. In 6 Monaten nimmt die Aktivität nicht mehr als um 5 % ab.

Beispiel 9

Natrium-Cephalosporin C (40 mM) wird bei pH 7,3 in 20 mM Kaliumphosphatpuffer gelöst und unter Rühren auf 30 °C temperiert und mit Sauerstoff begast. Dazu werden 2 % (w/w) immobilisierte DAO gegeben und der Ansatz mittels eines Autotitrators auf Anfangs-pH gehalten. Nach Beendigung der Reaktion wird die Lösung abgelassen und der Reaktor neu befüllt. Das Enzym verbleibt im Gefäß. Die Zahl der Umsetzungen beträgt 120. Die Reaktionszeit wird so gewählt, daß vollständiger Umsatz möglich ist. Sie beträgt bis etwa 40 Umsetzungen 1 Stunde, dann 1,5 bis zu 90 und schließlich 2 h.

Tabelle 1

| Vergleich der Enzymträger | | | |
|---|---|---|---|
| | Bindungseffizienz [$\eta$] | U/g (FGW) | $t_d$ bei 30 °, pH = 7,3 |
| Eupergit (Röhm) | 0,71 | 29 | χ 20 |
| VA-Epoxy (Riedel-deHaen) | 0,83 | 32 | >40 |
| Vinyl-Sepharose (Kem-en-tec) | 0,51 | 22 | χ 15 |
| BrCN-akt. Sepharose (Pharmacia) | 0,7 | 6 | ≈ 6 |

## Patentansprüche

1. Mit Enzymen beschichtetes Trägermaterial, das D-Aminosäureoxidase und ein poröses, perlförmiges Trägermaterial enthält, wobei das Trägermaterial aus einem vernetzten Copolymerisat, das im wesentlichen aus Vinylacetat- und/oder Vinylalkohol-Einheiten und Einheiten eines Vernetzungsmittels besteht, wobei die Einheiten des Vernetzungsmittels einpolymerisierte Verbindungen der allgemeinen Formeln

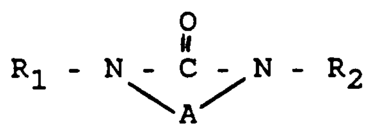

(I)

und/oder

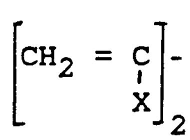

(II)

sind, wobei $R_1$, $R_2$ in Formel (1) gleich oder verschieden sein können und Vinyl-, 1-Acyloxy-Vinyl-, Allyl- oder 2-Acyloxy-Allyl- bedeuten, A einen zweiwertigen Kohlenwasserstoffrest mit 2 bis 8 C-Atomen darstellt, B in Formel (II) für einen zweiwertigen Kohlenwasserstoffrest mit 1 bis 8 C-Atomen steht und X Acyloxy bedeutet, wobei die Acyloxygruppe ein Rest mit 2 bis 18 C-Atomen ist, die Menge an Vernetzungsmittel-Einheiten 1 bis 60 Gew.-%, bezogen auf das Polymere, beträgt, und die Acyloxy-Gruppen der Vinylacylat-Einheiten als solche vorliegen oder teilweise oder vollständig zu Hydroxylgruppen verseift sind, und die mittlere Teilchengröße der Perlen 20 bis 800 µm und der mittlere Porendurchmesser 2 bis 10 000 nm beträgt.

2. Trägermaterial nach Anspruch 1, dadurch gekennzeichnet, daß die D-Aminosäureoxidase aus Hefen oder Pilzen stammt.

3. Trägermaterial nach Anspruch 1 oder 2, dadurch gekennzeichnet, daß es D-Aminosäureoxidase und Katalase enthält.

4. Verfahren zur Herstellung von beschichteten Trägermaterial nach Anspruch 1 oder 2, dadurch gekennzeichnet, daß eine D-Aminosäureoxidase-haltige Lösung mit einem porösen, perlförmigen Trägermaterial aus einem vernetzten Copolymerisat, das im wesentlichen aus Vinylacetat- und/oder Vinylalkohol-Einheiten und Einheiten eines Vernetzungsmittels besteht, wobei die Einheiten des Vernetzungsmittels einpolymerisierte Verbindungen der allgemeinen Formel

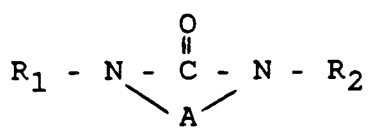

(I)

und/oder

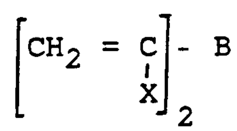

(II)

sind, wobei $R_1$, $R_2$ in Formel (1) gleich oder verschieden sein können und Vinyl-, 1-Acyloxy-Vinyl-, Allyl- oder 2-Acyloxy-Allyl- bedeuten, A einen zweiwertigen Kohlenwasserstoffrest mit 2 bis 8 C-Atomen darstellt, B in Formel (II) für einen zweiwertigen Kohlenwasserstoffrest mit 1 bis 8 C-Atomen steht und X Acyloxy bedeutet, wobei die Acyloxygruppe ein Rest mit 2 bis 18 C-Atomen ist, die Menge an Vernetzungsmittel-Einheiten 1 bis 60 Gew.-%, bezogen auf das Polymere, beträgt, und die Acyloxy-Gruppen der Vinylacylat-Einheiten als solche vorliegen oder teilweise oder vollständig zu Hydroxylgruppen verseift sind, und die mittlere Teilchengröße der Perlen 20 bis 800 µm und der mittlere Porendurchmesser 2 bis 10 000 nm beträgt, inkubiert wird.

5. Verfahren nach Anspruch 4, dadurch gekennzeichnet, daß eine aus Pilzen oder Hefen gewonnene D-Aminosäureoxidase-haltige Lösung verwendet wird.

6. Verfahren nach Anspruch 4 oder 5, dadurch gekennzeichnet, daß eine durch Ionenaustauscherchromatographie gereinigte D-Aminosäureoxidase-haltige Lösung verwendet wird.

7. Verfahren nach einem oder mehreren der Ansprüche 4 bis 6, dadurch gekennzeichnet, daß D-Aminosäureoxidase- und Katalase-haltige Lösungen verwendet werden.

8. Verfahren nach einem oder mehreren der Ansprüche 3 bis 7, dadurch gekennzeichnet, daß die Inkubation bei einer Temperatur zwischen 0 °C und +40 °C erfolgt.

9. Verfahren zur Herstellung von Glutaryl-7-aminocephalosporansäurederivaten oder $\alpha$-Ketoadipinyl-7-aminocephalosporansäurederivaten, dadurch gekennzeichnet, daß ein beschichtetes Trägermaterial nach einem oder mehreren der Ansprüche 1 bis 3 und Cephalosporin-Derivate eingesetzt werden.

**Patentansprüche für folgende Vertragsstaaten : ES, GR**

1. Verfahren zur Herstellung von beschichteten Trägermaterial, dadurch gekennzeichnet, daß eine D-Aminosäureoxidase-haltige Lösung mit einem porösen, perlförmigen Trägermaterial aus einem vernetzten Copolymerisat, das im wesentlichen aus Vinylacetat- und/oder Vinylalkohol-Einheiten und Einheiten eines Vernetzungsmittels besteht, wobei die Einheiten des Vernetzungsmittels einpolymerisierte Verbindungen der allgemeinen Formel

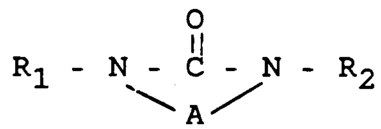

(I)

und/oder

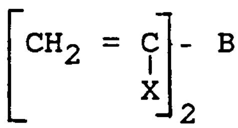

(II)

sind, wobei $R_1$, $R_2$ in Formel (1) gleich oder verschieden sein können und Vinyl-, 1-Acyloxy-Vinyl-, Allyl- oder 2-Acyloxy-Allyl- bedeuten, A einen zweiwertigen Kohlenwasserstoffrest mit 2 bis 8 C-Atomen darstellt, B in Formel (II) für einen zweiwertigen Kohlenwasserstoffrest mit 1 bis 8 C-Atomen steht und X Acyloxy bedeutet, wobei die Acyloxygruppe ein Rest mit 2 bis 18 C-Atomen ist, die Menge an Vernetzungsmittel-Einheiten 1 bis 60 Gew.-%, bezogen auf das Polymere, beträgt, und die Acyloxy-Gruppen der Vinylacylat-Einheiten als solche vorliegen oder teilweise oder vollständig zu Hydroxylgruppen verseift sind, und die mittlere Teilchengröße der Perlen 20 bis 800 µm und der mittlere Porendurchmesser 2 bis 10 000 nm beträgt, inkubiert wird.

2. Verfahren nach Anspruch 1, dadurch gekennzeichnet, daß eine aus Pilzen oder Hefen gewonnene D-Aminosäureoxidase-haltige Lösung von Pilzen verwendet wird.

3. Verfahren nach Anspruch 1 oder 2, dadurch gekennzeichnet, daß eine durch Ionenaustauscherchromatographie gereinigte D-Aminosäureoxidase-haltige Lösung verwendet wird.

4. Verfahren nach einem oder mehreren der Ansprüche 1 bis 3, dadurch gekennzeichnet, daß D-Aminosäureoxidase- und Katalase-haltige Lösungen verwendet werden.

5. Verfahren nach einem oder mehreren der Ansprüche 1 bis 4, dadurch gekennzeichnet, daß die Inkubation bei einer Temperatur zwischen 0 °C und +40 °C erfolgt.

6. Verfahren zur Herstellung von Glutaryl-7-aminocephalosporansäurederivaten oder α-Ketoadipinyl-7-aminocephalosporansäurederivaten, dadurch gekennzeichnet, daß ein beschichtetes Trägermaterial nach einem oder mehreren der Ansprüche 1 bis 5 und Cephalosporin-Derivate eingesetzt werden.